Europäisches Patentamt

European Patent Office

Office européen des brevets

(19)

(11) Publication number: **0 045 493**
**A2**

(12) **EUROPEAN PATENT APPLICATION**

(21) Application number: **81106007.8**

(22) Date of filing: **30.07.81**

(51) Int. Cl.³: **A 61 K 7/48**

(30) Priority: **31.07.80 US 174051**

(43) Date of publication of application:
**10.02.82 Bulletin 82/6**

(84) Designated Contracting States:
**BE DE FR GB IT**

(71) Applicant: **SYNTEX (U.S.A.) INC.**
**3401 Hillview Avenue**
**Palo Alto, California 94304(US)**

(72) Inventor: **Lindenthal, Margaret**
**1417 Villege Court**
**Mountain View Calif.94040(US)**

(72) Inventor: **Edelstein, Herb**
**1178 Hyde Avenue**
**San Jose Calif.95129(US)**

(74) Representative: **Schmied-Kowarzik, Volker, Dr. et al,**
**Siegfriedstrasse 8**
**D-8000 München 40(DE)**

(54) **Composition useful for preparing face masks and process preparing such composition.**

(57) A composition which forms a gel when mixed with water and is suitable for use as a face mask, the composition being comprised of a water soluble alginate material, a water soluble, bivalent metal salt reactor, a phosphate retarder, a suitable extender and an anionic surfactant.

EP 0 045 493 A2

Croydon Printing Company Ltd.

-1-

# FACE MASK USING ALGINATES

## BACKGROUND OF THE INVENTION

### Field of the Invention

This invention relates generally to cosmetic compositions which are used as face masks. More particularly it relates to an improvement in face masks which are prepared by using alginates wherein a small amount of an anionic surfactant is included in the alginate composition to improve its characteristics.

### Prior Art

Masks are an old method for treating facial skin. Their use in Egypt under the Pharaohs is speculated and their use can definitely be traced back to the Greeks and Romans. In the modern world eastern cultures, particularly the Japanese, have become the leaders in the use of this type of facial skin treatment both in the frequency of use and the technology involved.

Various materials are used for this type of treatment ranging from the simple mud pack to specifically formulated creams, jellies, powders and foams, removable by washing, to jellies and pastes which set to a gel-like consistency and then are removed by peeling the gel from the face after an appropriate amount of time.

The efficacy of masks for treating skin can be said to lie in three areas. One aspect is to increase the physiological function of the skin by shutting off normal skin respiration thereby raising skin temperature and moisture content. This increased temperature and moisture also has a secondary effect which is to enhance transfer of active ingredients in the pack to the skin via osmosis. Secondly, skin debris and other impurities may adhere to the mask material and be removed from the skin along with the mask, thus achieving a cleansing effect. Exfoliation of dry skin and rehydration may be achieved; and skin infection treated both through removal of debris and the transfer of medications to the skin. Finally, masks may also have a tightening effect on the skin, which in combination with a change in skin temperature, imparts a transient stimulating feeling of rejuvenated skin. (see Tonooka, N. and Tatsuya, I., Cosmetics and Toiletries, Vol. 95, p. 22-28, Feb., 1980.)

Mask compositions, except for mud packs which are suspensions, are in main based on hydrophilic colloid forming materials. The term colloid denotes a state of matter wherein particles are dispersed in some medium, the particles being of greater size than an ordinary crystalloid molecule but not large enough to settle out under the influence of gravity. Collodial particles usually vary in size from 10 to 5000 angstroms; although this size range is not the sole criteria necessary for formation of a colloid. The medium, or continuous phase, may be gas, liquid or a solid. When the medium is a liquid, the colloidal system is called a sol. A sol containing a substantial amount of liquid and which takes on a firm consistency is called a gel. When the prefix hydro is used, it denotes a sol or gel in which the liquid is water. A hydrophilic colloidial material is one capable of forming a sol or gel in water. The curing process by which a sol becomes a gel is called gelation,

4348S                                                               21860

a process which may be reversed, or, if the material becomes chemically cross-linked, irreversible.

Various inorganic clays and powders, as well as polar group containing organic compounds, are capable of forming hydrocolloids. For example, hectorite clays, kaolin, talc, zinc oxide and inorganic silicates, to name a few, disperse readily in water to give a gel when appropriate amounts of water and solid are employed. Numerous water soluble organic compounds of high molecular weight also form hydrocolloids, and are well known in the art. Generally this group includes: water soluble polysaccharides; proteins; poly-alcohols; imines; acrylamides; pyrrolidones; carboxylic polymers; their esters and alkali metal salts; and sulfonated polymers. (see U.S. Pat. 4,087,555).

All hydrophilic colloidal materials form reversible gels when properly formulated. Some can form irreversible gels, provided a cross-linking agent is incorporated in the composition. The two most commonly known such materials are a polysaccharide called algin, named after its source brown seaweed (algae), and polyvinyl alcohol (PVA). Algin, is considered to be a linear polymer of anhydro-beta-d-mannuronic acid. E. W. Skinner, Skinner's Science of Dental Materials, 7th Ed., (1973, Saunders, Pittsburg, PA.) p. 114. When a bivalent metal ion is added to a sol made of alginic acid, or a salt thereof, the bivalent ion reacts with the acid functionality of two alginate polymers forming an insoluble, irreversible alginate gel.

For these algin compositions to be effective as a face mask, several criteria must be met. It should have a smooth consistency, free of lumpiness or grittiness. Drying should occur rapidly once prepared and applied to the face, but not so quickly as to interfere with its application. The mask should be properly formed within

-4-

ca. 10 minutes from time of application. The dried mask should be easily and painlessly removable. A sensation of tightening of the skin should occur after application. Cleansing of the skin should occur while at the same time the mask must be dermatologically innocuous and non-toxic.

Present technology in the area of peelable masks faces several problems. So-called film-type masks, really reversible hydrocolloids, often require 20-30 minutes of curing time to gel properly. Materials which do gel in an acceptable time frequently adhere too tightly to the skin creating excessive tension on the skin or discomfort when removed. Certain formulations based on alginate gel technology may address, to some degree, these problems, but such gels are not always sufficiently smooth or flexible to be acceptable for use as a face mask and frequently the gelation time is so rapid, manipulation of the material before it gels is difficult.

## SUMMARY OF THE INVENTION

This invention is directed toward a face mask which gels quickly but not before application to the face is complete. Also, the final gel is acceptably smooth and flexible and will not adhere so strongly to the skin that removal has adverse effects on the skin.

The desired results are achieved by modifying standard alginate compositions to include an anionic surfactant which serves to both regulate gelation and impart smooth, elastic consistency to the gel, a result heretofore not seen with alginate-based face masks and also not seen using certain cationic, non-ionic and amphoteric surfactants.

More precisely, the present invention is drawn to a composition which will form a irreversible hydrocolloid gel when mixed with water and includes alginic acid or a

4348S                                                        21860

salt thereof, a water-soluble, bivalent metal ion salt, a reactor, an extender and an anionic surfactant, which is the component responsible for the qualities of smoothness and flexibility, in addition to modifying gelation time. A gel is obtained by mixing water or an aqueous solution of the anionic surfactant with the dry ingredients to form a paste-like sol. This sol is then applied to the face by hand or other appropriate means, allowed to gel and, following an appropriate interval, peeled from the face. These and other aspects of the invention are set forth hereinafter.

## DESCRIPTION OF THE INVENTION

Formulation of a workable composition for a gel as disclosed herein requires the preparation of a dry mixture comprised of a hydrophilic colloid forming material capable of forming an insoluble precipitate, a cross-linking, precipitating agent, a retarder to control the gelation reaction, extenders to facilitate blending and modify the gel texture and an anionic surfactant which serves to modify gelation time and impart smoothness to the gel. To form a gel, an appropriate amount of water is added to a premeasured amount of dry mix creating a paste-like sol which is applied to the face using the hand or some other appropriate means. An alternative method for creating the sol is to predilute the anionic surfactant with water, and mix that solution in an appropriate amount with an aliquot of the other premixed dry materials.

The hydrophilic colloid forming material used herein is an alginate, i.e. any water soluble form of algin, the polysaccaride material obtained from brown seaweed known as algae. Said alginate includes alginic acid and any of its known water soluble salts. An alginate or combination of alginates would comprise, by weight, about 10-30% of the dry mix as formulated herein.

4348S                                                           21860

Water soluble alginates having utility in this invention are alginic acid, its alkali metal salts, ammonium salts and quaternary amine salts. An alginate salt such as sodium, potassium, ammonium and triethanolamine alginate or mixtures thereof, present in an amount of 12-25% by weight of the dry mix is preferred, but most preferred is the sodium salt in an amount of 14-16%.

Formation of the irreversible gel requires a chemical agent, or reactor, which can serve both to combine individual alginate chains and form an insoluble precipitate. A water soluble, bivalent metal ion salt is the agent of choice herein. In preparing the dry mix the salt is added in a proportion by weight which parallels the amount of alginate in the mix. Generally, the alginate and the metal ion salt are present in the same amount. Any water soluble bivalent metal ion salt may be used but salts of calcium, magnesium or zinc are preferred. While these bivalent metal ions may be derived from an inorganic or organic salt, an inorganic source is preferred.

While an adequate gel will form when the salt is present in an amount ranging from 60 to 120% of the alginate's dry weight, a one to one ratio of water soluble, bivalent metal ion salt to alginate provides the most acceptable sol and gel. Therefore, said salt may be present in a weight/weight percent of between 10-30% but in the preferred formulation it is present in an amount of 12-25%, (and most preferably 14-16%), the same amount as noted above for the alginate.

The retarder is a basic salt which is added for the purpose of controlling, that is delaying, the gelation process, and, in so doing, modifying the texture and elasticity of the gel formed. Simple mixtures of alginate, bivalent metal ion and water gel very rapidly to a lumpy, gritty consistency with little elasticity. Addition of the basic salt which can exchange cation with the reactor to form a salt which is less soluble in water than the first salt, but whose anion does not contribute to the cross-linking reaction, serves to delay gelation and the extent of gelation. In so doing, the retarder gives a smoother, more elastic gel. The type of retarder commonly known in the art is inorganic and has a cation different from the soluble, bivalent metal ion salt. Most commonly used are alkali metal phosphates. Organic chelating agents have also been used as retarders, for example, sodium citrate, sodium carbonate and sodium oxalate. In the composition disclosed herein the primary retarder is a phosphate salt which comprises, by weight, about 2.5 to 15% of the dry mix. Any phosphate having monovalent alkali metal cations is acceptable. For example, trisodium phosphate, sodium hexametaphosphate, tripotassium phosphate, tetrasodium pyrophosphate, sodium tripolyphosphate and potassium tripolyphosphate, to name several examples, may be used to achieve satisfactory gels. Any amount ranging between 3 and 10% by weight is preferred to form a gel. It is most preferred to use tetrasodium pyrophosphate in an amount of 5-6% by weight.

A fourth component is a water soluble anionic surfactant. It is included in the composition for two purposes. First it has the capability of mediating gelation time. Secondly, the anionic surfactant facilitates rapid, even dispersion and solution of the solids into the water phase to give a gel with enhanced smoothness and flexibility. Numerous anionic surfactants of various carbon chain lengths having various acid functionalities, and their salts, are known. Any length carbon chain, saturated or unsaturated straight or branched, or otherwise substituted with, for example, phenyl, and having an acid group joined to the carbon chain directly or by means of an ether, ester, amine or amidine linkage may be used to practice this invention. Because the salts have greater water solubility they are preferred. The composition set out herein utilizes a sulfur derived anionic surfactant which is added in an amount of about 0.40% to 18.8% by weight in the dry mix or alternatively prepared as an aqueous solution in a concentration of about 0.10% to 5.0% weight/weight. A sulfur derived anionic surfactant should be understood to mean a surfactant having a sulfonate or sulfate anion which is bonded directly, or by ether linkage to a carbon chain having 8 to 22 carbon atoms or a salt thereof. The preferred sulfur derived anionic surfactants used to prepare sols and gels herein are the sodium, potassium, ammonium or triethanolamine salts of lauryl sulfate, lauryl ether sulfate or combinations thereof; 1-10% by weight/weight preferred for said dry mix or 0.5-2.0% weight/weight preferred for said aqueous solution. Sodium lauryl sulfate in an amount of about 6.25% weight/weight for the dry mix or 1% weight/weight for the aqueous solution, whichever is appropriate to the method of forming a sol, is most preferred.

Extenders are added to the dry composition to facilitate mixing of wet and dry materials, to modify gel texture, and to enhance the dimensional stability of the gel.

These extenders may consist of silicates, clays or inorganic salts. The quantity of extender added is a function of the amount of other dry ingredients. After all other dry ingredients have been measured out on a weight percent basis a quantity of extender sufficient to achieve 100% or unity, is added to the composition. In certain instances this may be as much as 80% of the dry composition.

Any extender known in the art may be used, for example, silica, magnesium carbonate, zinc oxide, talc, bentonite, kaolin, alumina oxide, magnesium silicate, magnesium trisilicate, diatomaceous earth or combinations thereof, but a preferred formulation of the dry mix would include magnesium carbonate, diatomaceous earth or talc. Most preferred is an equal quantity by weight of magnesium carbonate and talc in a quantity sufficient to bring the percentage composition of the dry mix to unity.

A proper working of this invention requires that all solid materials be dissolved or dispersed in an appropriate solvent which may be water or water in combination with some water miscible dermatologically innocuous organic solvent; for example, an alcohol, glycol, amine or highly dielectric aprotic solvent such as DMSO, and combinations thereof. Water alone is the preferred solvent.

The source, type and content of the water is of little concern in so long as it is potable. It may be deionized, softened, or hard water or the ion content may be otherwise regulated or manipulated. It may be sterilized if desired by any conventional means, for example, heat, filtration or chemical treatment but such is not mandatory. Temperature may vary between 15°-30°C. Because of gelation time considerations and user convenience and comfort, it is preferred to use ambient (generally 20-25°C) tap water. If the anionic surfactant is predissolved in water the same considerations and preferences hold for the water used in preparing the solution. It is preferred to use the prepared surfactant solution when it is at room temperature.

Solvent and dry mix must be combined in particular amounts, first to achieve an acceptable sol and gel, and secondly to provide a sufficient quantity for properly covering the intended area of treatment. Mixing, ease of application and gelation time dictate that for every unit by weight of dry mix there be added about 2.8-4.7 volumes of solvent, solvent meaning either water or aqueous anionic surfactant solution. It should be understood that when an anionic surfactant solution is used no surfactant is present in the dry mix. A preferred dry mix/solvent ratio would be one part dry mix to 3.4-4.1 parts solvent. Most preferred is a dry mix/solvent ratio of 1:3.75. In practice these ratios translate into, e.g., 16 grams of dry mix being combined with 45-70 ml in the first instance, 55-65 ml of solvent in the second instance and most preferred is 16 grams of dry mix combined with 60 ml of solvent.

To one skilled in the art of formulations it would be apparent that various combinations of the dry materials may be predissolved in the diluent water. Any or all of the soluble metal ion salt or extender, in addition to the anionic surfactant can be treated in this manner. As noted above, however, only the anionic surfactant has been chosen for such treatment.

The recitation of six components as outlined above should not be interpreted to mean other chemicals may not be included or that their inclusion may adversely affect the sol or gel. Where desired, trace amounts of, for example, preservatives, colorants, indicators, perfumes, rubefacients, or drugs may be incorporated into the dry mix or pre-dissolved in the diluent water.

A complete practice of this invention would involve weighing out all solid ingredients and combining them in no particular order in an appropriate mixing apparatus and thoroughly blending them. Pulverization of individual ingredients before mixing or further manipulation of the blended powder may be carried out at the discretion of the practitioner. A chosen volume of diluent is accurately measured into a mixing container which may be a bowl, shallow dish or the like and 16 grams of powder added with spatulation. Mixing is continued for approximately 1 minute, giving a paste-like sol. This sol is transferred immediately to the skin by means of the hand and fingers or some flexible, non-injurious instrument of the practioner's choosing. Metal or metal containing devices should not be used. The sol is cured to a gel by ambient temperature and airflow. If the dry mix is properly formulated and the solvent and powder well mixed, the resultant sol will undergo gelation to form a smooth, elastic gel within 8 to 15 minutes from the time mixing was begun. The gel may be allowed to remain on the face for any amount of time, but 20 minutes is the recommended treatment time. A properly formed gel is removed by grasping a leading edge of the gel and applying steady, even pressure to peel it from the face.

Where the anionic surfactant is pre-dissolved in the water the procedures outlined above, are repeated eliminating the surfactant from the dry mix.

Following are a number of examples setting out various combinations of components for practicing this invention.

-12-

## EXAMPLE I

A mask utilizing the preferred ranges of weights for each of the above-discussed components was prepared in the amounts set out below.

| Dry components | wt. % |
|---|---|
| sodium alginate | 15.6 % |
| Calcium Sulfate . $2H_2O$ | 15.6 |
| Tetrasodium pyrophosphate | 5.6 |
| Magnesium Carbonate | 31.3 |
| Lo-Micron Talc | 31.2 |
| Sodium Lauryl Sulfate | 0.7 |
| Total | 100.0 % |

All ingredients were weighed and mixed dry. Sixteen grams were then added to 60 cc of 25°C tap water and mixed for 1 minute spread on a surface and allowed to gel at ambient temperature without increased air flow. Gelation was declared complete when an inserted wooden toothpick came out clean. The paste resulting from this mix was smooth and creamy, easily applied to the surface and yielded a smooth non-gritty film gelling in 13 minutes which peeled readily.

## EXAMPLE II

Dry ingredients, except for the anionic surfactant, were weighed and blended in no particular order in the amounts designated in the chart.

| Dry components | wt. % |
|---|---|
| Sodium Alginate | 15.6 % |
| Calcium Sulfate dihydrate | 15.6 |
| Tetrasodium Pyrophosphate | 5.6 |
| Diatomaceous Earth | 63.2 |
| Total | 100.0 % |

Sixteen grams of this mixture were added to 60 ml of aqueous solution having a 1% concentration of sodium lauryl sulfate, mixed for 1 minute and spread on a smooth

4348S

21860

0045493

-13-

surface. Nine minutes were required to form a smooth, elastic gel which peeled easily.

EXAMPLE III

| Dry Ingredients | wt. % |
| --- | --- |
| Potassium Alginate | 15.63 % |
| Tetrasodium Pyrophosphate | 5.63 |
| Calcium Sulfate dihydrate | 15.63 |
| Magnesium Carbonate | 31.86 |
| Talc | 31.25 |
| Total | 100.00 % |

A 16 gram aliquot of this mixture was spatulated into 60 ml. of a 1% aqueous sodium lauryl sulfate solution for approximately 1 minute. The resulting sol was lump free and easily spread on a surface. Gelation occurred within 10 minutes. The gel was smooth, unmottled and easily lifted from the contacted surface.

EXAMPLE IV

| Ingredients | wt. % |
| --- | --- |
| Sodium alginate | 21.9 % |
| Tetrasodium Pyrophosphate | 8.1 |
| Calcium Sulfate Dihydrate | 21.9 |
| Magnesium Carbonate | 24.0 |
| Lo-Micron Talc | 24.1 |
| Total | 100.0 % |

Sixteen grams were spatulated into 60 ml of 1% sodium lauryl sulfate in water for 1 minute giving a slightly mottled solution which gelled in 8 minutes to a smooth easily lifted gel.

4348S                                                                 21860

-14-

## EXAMPLE V

| Ingredients | wt. % |
|---|---|
| Sodium alginate | 15.60 % |
| Tetrasodium Pyrophosphate | 3.80 |
| Calcium Sulfate Dihydrate | 15.60 |
| Magnesium Carbonate | 32.50 |
| Lo-Micron Talc | 32.50 |
| Total | 100.0 % |

Sixteen grams of this mixture dispersed in 60 ml of 1% aqueous sodium lauryl sulfate gave a slightly mottled sol which gelled in 8 minutes to give a smooth easily lifted gel.

## EXAMPLE VI

| Ingredients | wt. % |
|---|---|
| Sodium alginate | 15.63 % |
| Tetrasodium Pyrophosphate | 5.63 |
| Calcium Sulfate Dihydrate | 15.63 |
| Magnesium Carbonate | 31.86 |
| Lo-Micron Talc | 31.25 |
| Total | 100.0 % |

Sixteen grams of this mix were spatulated into 60 ml of a 0.17% aqueous sodium lauryl sulfate solution. The resulting sol and gel were smooth. Gelation occurred within 9 minutes giving a smooth, easily peeled gel.

## EXAMPLE VII

| Ingredients | wt. % |
|---|---|
| Sodium alginate | 15.63 % |
| Tetrasodium Pyrophosphate | 5.63 |
| Calcium Sulfate Dihydrate | 15.63 |
| Magnesium Carbonate | 31.86 |
| Lo-Micron Talc | 31.25 |
| Total | 100.0 % |

Into 60 ml of 2% aqueous sodium lauryl sulfate

-15-

solution was dispersed 16 g of noted dry mix. A smooth sol was obtained which gelled in ca. 9 minutes and was easily peeled from the contacted surface.

## EXAMPLE VIII

| Ingredients | wt. % |
|---|---|
| Sodium alginate | 30.0 % |
| Tetrasodium Pyrophosphate | 30.0 |
| Calcium Sulfate Dihydrate | 15.0 |
| Magnesium Carbonate | 12.5 |
| Lo-Micron Talc | 12.5 |
| Total | 100.0 % |

Sixteen grams of this mixture dispersed in 60 ml of 1% aqueous sodium lauryl sulfate gave a slightly mottled sol which gelled in 8 minutes to give a smooth easily lifted gel.

## EXAMPLE IX

As noted above, representative compounds from all four surfactant types were investigated to determine which was most efficacious. Table I below sets out the results of these experiments, illustrating that only anionic surfactants were found to have a salutory affect on either texture and elasticity or curing time.

An alginate composition containing 15.6% sodium alginate, 15.6% calcium sulfate dihydrate, 5.6% tetrasodium pyrophosphate, the remainder being made up of diatomaceous earth, was prepared as the standard mix. Sixteen grams of this mix were spatulated into 60 ml of various aqueous surfactant solutions containing 0.17-2% weight/weight for 45 minutes. The texture of the sol was noted. This sol was then spread on a smooth surface to cure. When an inserted toothpick came out cleanly gelation was declared complete. Gel texture, elasticity and peelability were then noted. A composition was

4348S                                                            21860

-16-

declared acceptable if it formed a lump-free sol, gelled in approximately 8-15 minutes to form a smooth, lump and grit-free gell which did not adhere strongly to the contacted surface and lifted easily and completely. Those compositions considered usable for a face mask are labeled "A" and those not meeting one or more stated criteria are labeled "U" or unacceptable.

4348S                                                                      21860

## TABLE I

| Add 16 g of Powder to 60 cc of each of the following solutions. | w/w % in deionized water | TIME | | APPEARANCE | | PEELABILITY | ACCEPTABILITY |
|---|---|---|---|---|---|---|---|
| | | MIX (sec) | GEL (min) | PASTE | FILM | | |
| Sodium Lauryl Sulfate | 0.17 | 45 | 9.00 | smooth | smooth | OK | A |
| Sodium Lauryl Sulfate | 1.00 | 45 | 9.25 | smooth | smooth | OK | A |
| Sodium Lauryl Sulfate | 2.00 | 45 | 8.75 | smooth | smooth | OK | A |
| Sodium Lauryl Ether Sulfate | 1.00 | 45 | 7.13 | smooth | smooth | OK | A |
| Ammonium Lauryl Sulfate | 1.00 | 45 | 12.42 | smooth | smooth | OK | A |
| Ammonium Lauryl Ether Sulfate | 1.00 | 45 | 9.75 | smooth | smooth | OK | A |
| Triethanolamine Lauryl Sulfate | 1.00 | 45 | 10.17 | extra smooth | smooth | OK | A |

-17-

| | | | | | | |
|---|---|---|---|---|---|---|
| Lauric Myristic Diethanolamide | 1.00 | 45 | 6.00 | smooth | smooth | OK | U |
| Alpha Olefin Sulfonate | 1.00 | 45 | 7.00 | smooth | smooth | OK | U |
| Disodium mono oleamido PEG2 Sulfosuccinate | 1.00 | 45 | 6.58 | smooth | smooth | OK | U |
| Sulfated Oleic Acid | 1.00 | 45 | 9.42 | smooth | smooth | OK | A |
| Sodium Ethylene Diamine Tetracetic Acid | 1.00 | 45 | 9.17 | very lumpy | lumpy | OK | U |
| Amphoteric-2 | 1.00 | 45 | 4.08 | smooth | smooth | OK | U |
| Sodium N Methyl N Coconut Acid Taurate | 1.00 | 45 | 6.33 | slightly lumpy | smooth | OK | U |
| Cocamido Propyl Betaine | 1.00 | 45 | 6.25 | smooth | smooth | OK | U |
| Sodium Trideceth 7 Carboxylate | 1.00 | 45 | 6.08 | smooth | smooth | OK | U |

4348S

21860

0045493

| | | | | | slightly | | |
|---|---|---|---|---|---|---|---|
| Dimethyl Difatty Ammonium Chloride | 1.00 | 45 | 6.67 | lumpy | mottled | OK | U |
| Alkylamidoimadazolinium Methyl methosulfate | 1.00 | 45 | 6.50 | lumpy | slightly mottled | OK | U |
| Dimethyl Dihydrogenated Tallow Ammonium Chloride | 1.00 | 45 | 5.75 | slightly mottled | slightly mottled | OK | U |
| 100% Deionized Water | -- | 45 | 6.75 | slightly lumpy | smooth | OK | U |

0045493

The foregoing examples are but a few of the total possible formulations which may be developed using the basic ingredients and it should be understood the recitation of these compositions is not intended to limit in any way the scope of this invention.

## WHAT IS CLAIMED IS:

1. A dry composition which, when mixed with an appropriate amount of water, forms a paste that gels to a smooth, elastic consistency suitable for use as a removable face mask and which is comprised of:

    (a) a water soluble alginate material;

    (b) a water soluble, bivalent metal ion salt;

    (c) a retarder;

    (d) a water soluble anionic surfactant; and

    (e) a cosmetologically acceptable extender.

2. The composition of Claim 1 wherein:

    (a) said alginate material is present in an amount of 10-30% wt/wt, preferably 14-16% wt/wt;

    (b) said salt is present in an amount of 10-30% wt/wt, preferably 14-16% wt/wt;

    (c) said retarder is present in an amount of 2.5-15% wt/wt, preferably 4-6% wt/wt;

    (d) said anionic surfactant is present in an amount of 0.4-18.8% wt/wt, preferably 6.25% wt/wt; and

    (e) said extender is present in a quantity sufficient to bring the total composition weight to unity.

3. The composition of Claim 1 or 2 wherein:

    (a) said alginate material is alginic acid, sodium alginate, potassium alginate, ammonium alginate, or triethanolamine alginate;

    (b) said salt is a calcium, magnesium or zinc salt;

    (c) said retarder is trisodium phosphate, tripotassium phosphate, sodium hexametaphosphate, tetrasodium pyrophosphate, sodium tripolyphosphate, or potassium tripolyphosphate;

4348S                                                    21860

(d)    said anionic surfactant is the sodium, potassium, ammonium or triethanolamine salt of lauryl sulfate; and

(e)    said extender is silica, magnesium oxide, magnesium carbonate, zinc oxide, talc, bentonite, kaolin, alumina oxide, magnesium silicate, magnesium trisilicate, diatomaceous earth, or mixtures of two or more of the foregoing extenders.

4.    An article of manufacture which comprises two separate receptacles, one containing a dry composition and the other containing an aqueous solution, wherein said dry composition is comprised of:

(a)    a water soluble alginate material;

(b)    a water soluble, bivalent metal ion salt;

(c)    a retarder; and

(d)    a cosmetologically acceptable extender;

and said aqueous solution comprises a water soluble anionic surfactant and water, which upon combination with said dry composition, forms a sol which gels to a smooth, elastic consistency suitable for use as a removable face mask.

5.    The article of manufacture of Claim 4 wherein the dry composition comprises:

(a)    said alginate in an amount of 10-30% wt/wt, preferably 14-16% wt/wt;

(b)    said ion salt in an amount of 10-30% wt/wt, preferably 14-16% wt/wt;

(c)    said retarder in an amount of 2.5-15% wt/wt, preferably 4-6% wt/wt;

(d)    said extender in a quantity sufficient to bring the total composition weight to unity;

and the aqueous solution is comprised of 0.1-5.0% wt/wt, preferably 1%, of a water soluble anionic surfactant in

4348S                                                    21860

- 23 -

0045493

water, their being 2.8-4.7 units by volume of aqueous solution present for every unit by weight of dry composition.

6. The article of manufacture of Claim 4 or 5 wherein said dry composition is comprised of:

(a) a water soluble alginate material which is alginic acid, sodium alginate, potassium alginate, ammonium alginate, or triethanolamine alginate;

(b) a water soluble salt which is a calcium, magnesium or zinc salt;

(c) a retarder which is trisodium phosphate, tripotassium phosphate, sodium hexametaphosphate, tetra sodium pyrophosphate, sodium tripolyphosphate, or potassium tripolyphosphate; and

(d) an extender which is silica, magnesium oxide, magnesium carbonate, zinc oxide, talc, bentonite, kaolin, alumina oxide, magnesium silicate, magnesium trisilicate and diatomaceous earth, or mixtures thereof;

and said aqueous solution is comprised of a water soluble anionic surfactant which is sodium, potassium, ammonium, or triethanolamine lauryl sulfate and water.

7. A composition which is a smooth, elastic gel suitable for use as a removable face mask, said composition being prepared by mixing water with components (a), (b), (c), (d) and (e) of claim 1, 2 or 3.

8. A process for making a smooth, elastic gel composition wherein the process comprises mixing water with components (a), (b), (c), (d) and (e) of claim 1, 2 or 3.

4348S

21860

9.    A process for making a face mask which comprises mixing components (a), (b), (c), (d) and (e) of Claim 1, 2 or 3 with water to form a sol; applying the sol to the facial skin; and allowing the sol to cure to form a smooth, elastic, removable gel.

10.    The use of the smooth elastic gel composition obtained according to Claim 8 or 9 for use as a removable face mask.